# EUROPEAN PATENT APPLICATION

(11) **EP 1 386 926 A1**
(43) Date of publication of application: **04.02.2004**
(21) Application number: 02291918.7
(22) Date of filing: 29.07.2002
(51) Int. Cl.: C07K 1/13, C12N 15/86, C07C 391/00

(54) **Methods for producing labelled recombinant polypeptides and uses thereof**

(71) Applicant: Bioxtal, 59100 Roubaix (FR)
(72) Inventor: Lundstrom, Kenneth, 4104 Oberwil (CH); L'Hermite, Etienne, 59130 Lambersart (FR)
(74) Representative: Tezier Herman, Béatrice

(57) **Abstract**

The present invention relates to methods for producing labelled recombinant polypeptides and uses thereof. The present invention more particularly discloses efficient methods for producing labelled recombinant polypeptides in mammalian host cells using viral expression systems, particularly labelled membrane proteins. The invention also relates to cells, kits and other products for performing the above methods, which allow and greatly improve structural analysis of polypeptides and rational drug design.

## Description

### Field of Invention

The present invention relates generally to methods for producing labelled recombinant polypeptides and uses thereof. The present invention more particularly discloses efficient methods for producing labelled recombinant polypeptides in mammalian host cells using viral expression systems, particularly labelled membrane proteins. The invention also relates to cells, kits and other products for performing the above methods, which allow and greatly improve structural analysis of polypeptides and rational drug design.

### Background of the invention

Structural information of proteins has improved the understanding of protein function and biological mechanisms in general, but has recently also started to play a major role in rational drug design. The knowledge of the precise site of interaction between the drug and its receptor/target has enabled the engineering of more potent drugs with reduced side effects.

Obtaining structural information has, however, required long-term commitment in labor-intensive and time-consuming technologies. Recombinant protein expression, purification and crystallization are all procedures with limited success rates. Particularly, progress has been extremely modest in the field of membrane proteins.

X-ray crystallography is used to determine the three-dimensional structure of polypeptides, but it is often necessary to make iterative computer intensive calculations of repetitive X-ray diffraction patterns as to unambiguously identify specific regions of the polypeptide.

Heavy atoms, having an atomic mass of greater than 20, are often added to the polypeptide during crystallization in an attempt to provide a point of reference, since these atoms provide unique signature(s) in multiwavelength anomalous diffraction (MAD) and multiple isomorphous replacement (MIR) analysis. The heavy atoms are added to the crystal randomly, or at most or all locations of a certain amino acid throughout the polypeptide, e.g., a replacement of all methionines in a protein with a selenomethionine (Hendrickson et al., EMBO J., 9:1665-1672, 1990).

Membrane proteins are involved in a multitude of biological processes: the respiratory chain, photosynthesis, transport of solute molecules and ions and regulating cellular responses to a wide range of biological molecules such as hormones, neurotransmitters and drugs. High resolution structural data has allowed useful insight into the function of a number of integral membrane proteins including the G-protein coupled receptor (GPCR) bovine rhodopsin (Palczewski et al. (2000) Science Vol. 289, no 5480, p 739-745), the mitochondrial cytochrome bcl complex (Iwata et al. (1998) Science 281, 64-71), bacteriorhodopsin (Pebay-Peyroula et al. (1997) Science Vol. 277, no 5332, p 1676-1681) and cytochrome c oxidase (Iwata et al. (1995) Nature 376: 660, Tsukihara et al. (1996) Science 272, 1136-1144). Despite these successes, the number of resolved membrane protein structures remains extremely small compared with soluble proteins.

To date, of approximately 12,000 protein structures deposited in the Brookhaven Protein Database, only 20 are membrane proteins. Furthermore, of these 20, very few of them (less than five) are eukaryotic in origin (see for instance Iwata et al. (1998) Science 281,64-71; and Tsukihara et al. (1996) Science 272, 1136-1144). These are poor numbers in light of the fact that it is estimated that 35-40% of the genes within the human genome code for integral membrane or membrane-associated proteins. Low levels of endogenous expression, high hydrophobicity and low stability in solution all combine to frustrate the membrane protein crystallographer. In many cases, even if enough pure protein can be obtained, it is impossible to grow crystals suitable for structural analysis, if indeed, they grow at all. Although *Escherichia coli* vectors have a number of advantages over other expression systems, such as low costs and rapid production of high cell densities, many proteins fail to express in bacterial cells.

Progress in solving some of these problems has been made using fusion protein technology. Fusion of soluble proteins, such as maltose binding protein, to the hydrophobic neurotensin receptor, a GPCR, has been shown to increase expression and facilitate purification (Tucker & Grisshammer (1997) Biotechnol. Lett. Vol.19, no.5, p. 425-428). In addition, the crystallisation of cytochrome c oxidase was facilitated by the addition of a monoclonal Fv fragment (Istawa et al. (1995) Nature, vol. 376, no. 6542, p. 660-669). Alternatively, crystallisation in lipidic cubic phase has yielded a high-resolution structure of bacteriorhodopsin (Landau & Rosenbusch. (1996) Proc. Natl. Acad. Sci. USA, vol.93, no. 25, p. 14532-14535; Pebay-Peyroula et al. (1997) Science Vol. 277, no 5332, p 1676-1681).

However, widely applicable methods to obtain structural information more readily have yet to be described.

The feasibility of incorporation of selenomethionine into recombinantly expressed proteins has earlier been demonstrated for various expression systems. E. coil-based expression vectors have been employed to obtain selenomethionine-labeled recombinant proteins (Doublie, S. (1997) Meth. Enzymol. 276, 523-530). Moreover, selenomethionine incorporation has also been successfully achieved for recombinant proteins expressed from *Saccharomyces cerevisiae* vectors in yeast cells (Bushnell, D.A., Cramer, P. and Cornberg, R.D. (2001) Structure 9, 11-14) and in Baculovirus-infected insect cells (Bellizzi, J.J., Widom, J., Kemp, C.W. and Clardy, J. (1999) Structure Fold Des 7, 263-267). Crystal structures at a resolution of 2.25 Å of selenomethionine labeled bovine palmitoyl-protein thioesterase 1 (PPT1) was determined by using the multiwavelength anomalous diffraction phasing method (Bellizzi et al. (2000) Proc. Natl. Acad. Sci. USA 97, 4573-4578).

The possibility to incorporate selenomethionine into recombinant proteins expressed in mammalian cells would obviously be of great benefit. The advantage is the presence of the transport, folding and post-translational machinery required for mammalian proteins. However, the general obstacle thereof is the sensitivity of mammalian cells to manipulations and changes in the environmental circumstances, which has strongly limited the growth of cells under conditions required for incorporation of selenomethionine.

### Summary of the Invention

The present invention provides novel methods for producing labelled recombinant polypeptides in mammalian expression systems. The invention discloses that labelling of recombinant polypeptides by incorporation of labels, such as heavy metals, in their structure is possible in mammalian host cells, and can be performed very efficiently under particular conditions. In this regard, the inventors have discovered that efficient selenomethionine labelling of recombinant proteins expressed in mammalian host cells is possible by using specific viral vectors and/or culture conditions. This production of labelled recombinant proteins in mammalian host cells has direct impact on structural biology and rational drug design.

In a particular aspect, the present invention thus provides a process for the incorporation of selenomethionine into recombinant proteins expressed in mammalian cells.

In another aspect, the invention relates to a method of producing labelled recombinant polypeptides in mammalian host cells, particularly selenomethionine-labelled recombinant polypeptides.

In a further aspect, the present invention relates to methods of producing or determining the three-dimensional structure of a polypeptide, comprising (i) the production and labelling of said polypeptide in a mammalian host cell and (ii) the purification and crystallography of said polypeptide based on said label.

Further aspects of this invention include cell compositions and cultures as well as kits suitable for the performance of the above methods.

As will be disclosed in this application, the above methods use a viral vector (or a recombinant virus) and/or particular culture conditions to produce the recombinant, labelled polypeptide. Most preferred viruses are alphavirus vectors, which may be employed to infect various mammalian host cells or cell lines cultured either adherently or in suspension cultures.

The above systems and/or conditions enable efficient incorporation of selenomethionine into a nascent polypeptide, during a reasonably short time, long enough for the host cells to survive. Another advantage of the invention is the induction of apoptosis by alphaviruses shortly after infection, which results in a strong inhibition of host cell protein synthesis.

The produced selenomethionine-labelled proteins can be subjected to multiwavelength anomalous diffraction technologies to allow structural analysis of recombinant proteins and, subsequently, rational drug design.

The invention can be used to label and produce any desired polypeptide, such as membrane proteins or receptors. The invention is particularly advantageous since mammalian host cells maintain polypeptides in a biologically active conformation.

### Brief Description of The Figures

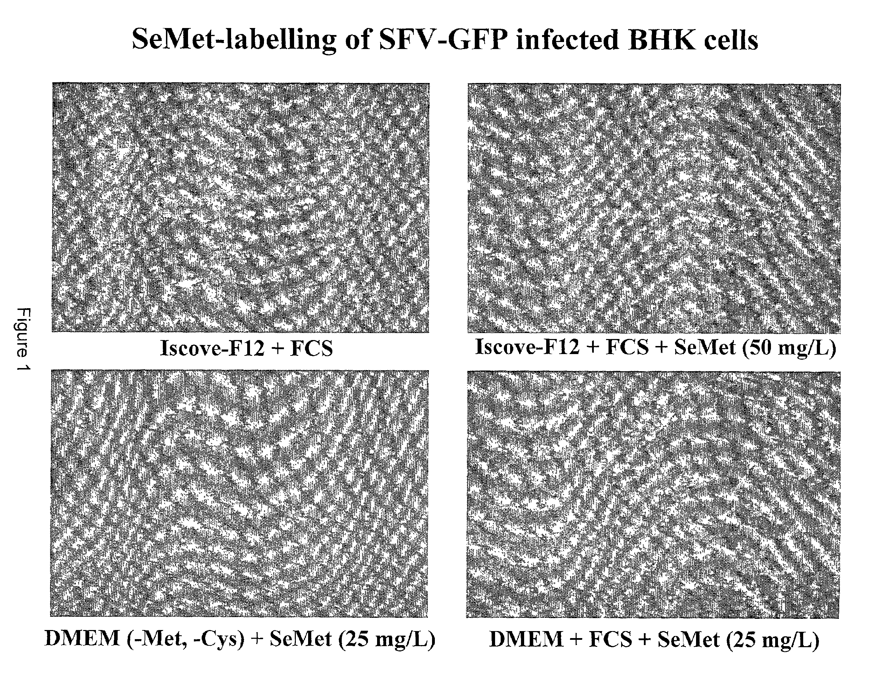

Figure 1: SFV-based expression of GFP in BHK-21 cells at 24 h post-infection in the absence (A) and presence (B) of selenomethionine.

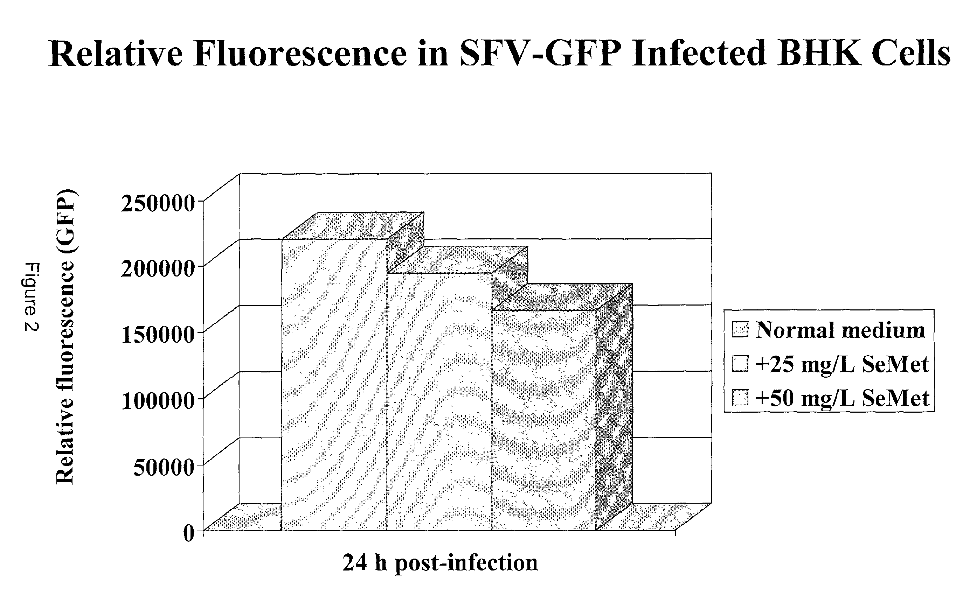

Figure 2: Comparison of GFP expression levels from SFV-infected cells cultured in normal medium or selenomethionine containing medium.
Figure 3: Expression levels of topologically different proteins in various mammalian host cell lines.

### Detailed description of the invention

The invention provides methods of producing labelled, recombinant polypeptides in mammalian host cells, particularly for the incorporation of labelled amino acid residues into nascent polypeptides in mammalian host cells. The invention is particulary suited to incorporate amino acids labelled with heavy metals, more specifically selenomethionine.

Labelled proteins include proteins comprising heavy atoms, i.e., elements with atomic numbers of at least 20, such as selenium, Hg, Pt, Au, U and Pb. Preferably, selenium, in particular in the form of selenomethionine, is the label used in the present invention.

The process of the present invention for producing labelled, recombinant polypeptides in mammalian host cells, in particular selenomethionine-labelled recombinant proteins, comprises the use of alphavirus or other specific viral expression vectors.

A particular object of this invention resides in a method for producing a selenomethionine labelled recombinant polypeptide, comprising : a) transfecting mammalian cells with an alphavirus or other specific viral vector comprising a nucleic acid sequence encoding a polypeptide, b) culturing the cells in a selenomethione-containing growth medium under conditions allowing incorporation of at least one selenomethionine residue into the polypeptide and, c) optionally, isolating the obtained polypeptide from the cell or the growth medium, said polypeptide being labelled.

In a specific embodiment, the process can further comprise the following steps: d) forming a crystal of the polypeptide; and e) performing X-ray crystallography on the crystal, wherein the incorporated label, in particular selenomethionine residue, is used to determine the structure of the polypeptide.

The present invention further provides a use of a recombinant vector, or a cell, according to the invention, in a method of obtaining structural data on a protein of interest according to the invention.

Unless defined otherwise, all technical terms and scientific terms used herein have the same meaning as commonly understood by one of ordinary skill in the art to which this invention belongs. Although any methods and materials similar or equivalent to those described herein can be used in the practice or testing of the present invention, the preferred methods and materials will now be described. All patents, patent applications, and publications mentioned herein are incorporated by reference. In addition, the materials, methods and examples are illustrative only and not intended to be limiting.

The method of the present invention uses an alphavirus vector or other specific viral expression vectors, to produce the recombinant polypeptide in mammalian host cells. The term "alphavirus vector" designates any nucleic acid molecule comprising a recombinant alphavirus genome which includes a target nucleic acid sequence encoding a selected polypeptide. The molecule may be a plasmid or a linear fragment, in the form of DNA or RNA, single- or double-stranded. The term "alphavirus vector" also includes recombinant alphaviruses, i.e., alphavirus particles comprising a nucleic acid molecule as described above. These definitions can also be applied to the other specific viral expression vectors used in the present invention.
The alphavirus vector may be of various origin, including Aura virus, Babanki virus, Barmah Forest virus, Bebaru virus, Buggy Creek virus, Chikungunya virus, Eastern Equine Encephalitis virus, Everglades virus, Fort Morgan virus, Getah virus, Highlands J virus, Kyzylagach virus, Mayaro virus, Middelburg virus, Mucambo virus, Ndumu virus, O'Nyong-Nyong virus, Pixuna virus, Ross River virus, Sagiyama virus, Semliki Forest virus, Sindbis virus, South African Arbovirus No. 86, Una virus, Venezuelan Equine Encephalitis virus, Western Equine Encephalitis virus and Whataroa virus.

The preferred alphavirus vectors are derived from Semliki Forest virus (SFV) (Liljeström and Garoff(1991) Bio/Technology 9, 1356-1361), Sindbis virus (SIN) (Xiong et al. (1989) Science 243, 1188-1191) or Venezuelan Equine Encephalitis virus (VEE) (Davis et al. (1989) Virology 171, 189-204), but does not exclude the use of other alphavirus vectors developed.

Additionally, other types of viral vectors including Adenovirus and Herpes simplex virus (HSV) can be applied. In a preferred embodiment, the viral expression vector is an alphavirus expression vector and more particularly the SFV.

The method can be used to produce any polypeptide of interest, i.e., any peptide, protein or fragment thereof, of any origin, typically of mammalian origin, including human origin. It is typically used to produce polypeptides for which the amino acid (or encoding nucleotide) sequence is known and for which structural data is desired. In a particular embodiment, the polypeptide of interest is a membrane protein, either a partial membrane protein or an integral membrane protein, such as a G-protein coupled receptor (GPCR). The integral membrane protein may have any number of transmembrane domains, ranging from one to twelve or more. Additionally, nuclear, cytoplasmic and secreted proteins can be the targets.

Methods of modifying the polypeptide to incorporate a selenomethionine residue are described herein.

In a first step, the process comprises transfecting a mammalian cell with an alphavirus or other specific viral vector comprising a nucleic acid sequence encoding the selected polypeptide. The term transfecting designates, generally, any method by which an alphavirus or other specific viral vector may be introduced into a mammalian cell, including transformation, transfection, infection, etc.

In a typical embodiment, the alphavirus vector is or comprises a nucleic acid molecule or construct which comprises the sequence of a recombinant alphavirus genome, said recombinant alphavirus genome (i) being deficient for at least one alphavirus structural protein coding gene, (ii) comprises alphavirus non-structural protein coding sequences and regulatory sequences, and (iii) comprises a nucleic acid sequence coding the polypeptide.

The structure and sequence of alphavirus genomes have been disclosed in the prior art (see for instance US Patent No. 5,739,026, which is incorporated therein by reference). Briefly, the genome comprises sequences encoding non-structural proteins (nsPs), sequences encoding structural proteins (e.g., the capsid, envelope proteins, etc), as well as regulatory sequences necessary for replication and packaging. More specifically, the non-structural proteins are encoded as a poly-precursor nsP1-4, which is then cleaved into four separate proteins. Similarly, the 26S RNA encodes all of the structural proteins of the virus, as a poly-precursor, namely : C, p62, 6K and E1. The main regulatory sequences are located at the terminal ends of the genome (typically in the first and last 250 nucleotides). Alphaviruses or alphavirus genomes or fragments thereof may be obtained by various techniques and from various sources. They may be synthesized artificially, cloned from plasmids or from virus isolates by RT-PCR, or derived or purified directly from virus samples deposited in libraries.

Particular alphavirus vectors according to this invention comprise a nucleic acid sequence encoding at least one, preferably all of the non-structural proteins nsP1-4 (including functional equivalents or derivatives thereof) and lack at least one functional nucleic acid encoding a structural alphavirus protein selected from C, E1, E2, E3 and 6K. The structural protein gene sequences may be rendered non functional either by mutation(s), insertions) or deletion(s), or a combination thereof, for instance. Preferably, the structural protein gene sequences are rendered non functional by deletion, i.e., they are absent from the recombinant alphavirus vector, in all or in part. Further preferred alphavirus vectors are thus devoid of nucleic acid encoding at least one, preferably all structural alphavirus proteins selected from C, E1, E2, E3 and 6K. Alphavirus vectors should, however, comprise the 5' and 3' regulatory sequences necessary for efficient viral replication and packaging.

A preferred recombinant alphavirus vector according to this invention designates a nucleic acid construct comprising the sequence of at least a 5'-terminal portion, a region coding for non-structural proteins and a 3'-terminal portion of an alphavirus genome, which are required for replication and packaging, said construct being devoid of any alphavirus structural protein coding sequence. The alphavirus vector may further comprise the sub-genomic promoter region, i.e., the 26S promoter, or a functional equivalent thereof. The vector may include regions that code for some alphavirus structural proteins, such as the translation enhancement signal located in C.

The recombinant alphavirus vectors comprise a target polynucleotide sequence, i.e., any polynucleotide sequence encoding the polypeptide whose labeling and production is desired. The polynucleotide may be located in various regions of the vector, as long as the target sequence does not prevent replication and/or packaging of the vector. Examples of such insertion sites include, typically, downstream of the non-structural coding sequences.

The total size or length of the recombinant alphavirus vector should be compatible for packaging into infectious alphavirus particles. More preferably, they should not exceed by more than 20% the size of a natural alphavirus genome.

The nucleic acid sequence encoding the polypeptide of interest may be heterologous or homologous. By "heterologous" nucleic acid is meant a nucleic acid, which is partly or entirely foreign to the mammalian cell or animal in which it is introduced.

The alphavirus expression vector comprises preferably at least part of an alphavirus genome and a nucleic acid sequence encoding the polypeptide to be selenomethionine labelled, inserted downstream of an alphavirus base sequence having translation enhancing activity. The alphavirus expression vector according to the invention is capable of efficient infection of animal host cells. The alphavirus RNA genome comprises the complete alphavirus RNA genome regions, which are essential to replication of the said alphavirus RNA. The nucleic acid sequence encoding the polypeptide of interest is preferably a heterologous RNA. Such vectors can thus be used to achieve enhanced levels of expression of DNA or cDNA coding for the polypeptide of interest after introduction of said vector in the mammalian cell. Specific methods of producing alphavirus expression vectors are also described in WO 92/10578 or in US patent N° 6,224,879.

In a particular embodiment, in the case of SFV, *in vitro* transcribed RNA is co-electroporated into baby hamster kidney (BHK-21) cells with RNA from the pSFV-Helper2 vector to generate high-titer replication-deficient particles (Berglund et al. (1993) Bio/Technology 11, 916-920). Chymotrypsin activation is required before the recombinant SFV particles are infectious for mammalian cells.

Various mammalian cells can be efficiently infected by alphavirus or other specific viral expression vectors, in particular SFV.

The cell population to be used in the invention comprises mammalian cells, such as blood cells, epithelial cells, endothelial cells, fibroblasts, hepatocytes, neurons, glial cells, etc. The cells are preferably established as cell lines, that can be cultured and stored. The cells can be of various mammalian origin, including human, rodent, bovine, porcine, canine, etc. Preferred cells are of human or rodent (e.g., murine, rat) origin. Typical examples of such cells include BHK-21, CHO-K1, HEK293 and C6 glioma cells. It should be understood that any other mammalian cell may be used in this invention.

The invention also relates to any population of mammalian cells, in particular any population of human, murine or rat cells, that comprises a viral vector expressing system as defined above. The invention describes the production of such cells, and demonstrates that they can be used for the production of selenomethionine-labelled polypeptides with efficacy, sensitivity and reproducibility.

For performing the instant invention, it is not necessary that pure cell populations be used. In particular, the cell population may comprise 20 % of cells or of cells that do not contain the expression vector. However, it is preferred to use cell populations that preferably comprise at least 80 % of cells expressing the polypeptide of interest and containing the viral vector in order to increase the efficacy and sensitivity of the method. More preferably, the cell populations comprise at least 85 % of these cells and most preferred at least 90 % .

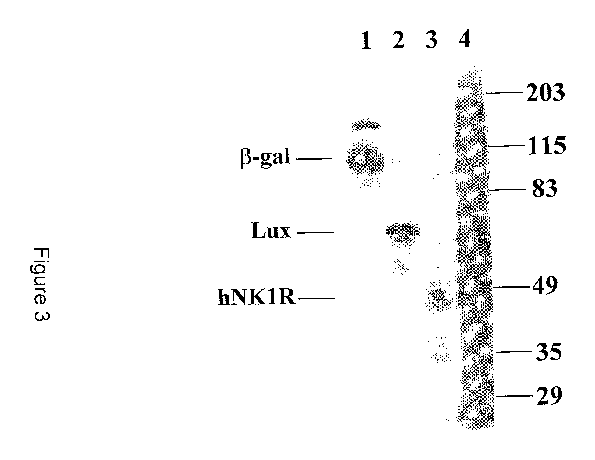

Generally, the alphavirus or other specific viral expression vectors, in particular SFV, confer a strong cytotoxicity to host cells within 24 h after infection. On the other hand, the expression of heterologous proteins is rapid and extremely efficient. Furthermore, metabolic labelling of mammalian cells infected with alphavirus or other specific viral expression vectors, in particular SFV-infected mammalian cells, with [³⁵S] methionine has indicated that short pulses of 15 minutes can efficiently incorporate the radioisotope in the recombinant protein synthesized in the host cells (Fig. 3). Additionally, alphavirus or other specific viral, in particular SFV, infection leads to strong inhibition of host cell protein synthesis, which can be demonstrated by an almost complete absence of expressed host cell proteins visualized by SDS-PAGE (Fig. 3). These features therefore allows for efficient labelling of recombinant proteins with selenomethionine using alphavirus or other specific viral gene delivery.

The selenomethione-containing growth medium typically contains between 1 and 100 mg/L, preferably 5 to 60 mg/L, and most preferably 7 to 55 mg/L of selenomethionine. The transfected cells can be maintained in any culture medium suitable for mammalian cells, including RPMI, DMEM, generally around 37°C (for human cells), supplemented with conventional additives (antibiotics, amino acids, serum, etc.). The cells can be cultured and/or stored in any appropriate device (tubes, flasks, bottles, etc.). Cell viability and/or absence of contamination can be verified prior to carrying out the methods of this invention.

In a specific embodiment, the present invention provides a kit for use in a method for producing labelled, recombinant polypeptides or for determining a polypeptde structure, comprising a mammalian cell and/or a recombinant vector and/or a selenomethionine-containing growth medium, as defined above.

The present invention further provides a use of a recombinant vector, a mammalian cell, or a selenomethionine-containing growth medium, as defined above, in a method for obtaining structural data on a protein of interest.

Incorporation of selenomethionine is evaluated by first subjecting various mammalian host cells to growth in medium containing selenomethionine. The viability of the SFV-infected and non-infected control cells is validated. It is expected that cells will survive long enough to enable efficient incorporation of selenomethionine into synthesized recombinant proteins.

Selenomethionine labeled proteins obtained according to the invention may be subjected to crystallization and subjected to multiwavelength anomalous diffraction methods for structure determinations.

In general, a polypeptide is suitable for crystallographic analysis by a method of the invention if: 1) the nucleotide sequence of the gene encoding the polypeptide is known, and 2) the selenomethionine-labelled polypeptide can be isolated and crystallized. A selenomethionine-labelled polypeptide can be isolated and crystallized by well-known techniques.
An "isolated polypeptide" as used herein refers to a polypeptide subjected to a procedure resulting in the purification to any degree relative to its naturally obtained state. The purified or isolated protein or peptide also includes molecules free from the environment in which they may naturally occur. Generally, purification or isolation refers to a protein or peptide subjected to fractionation to remove various other components without loosing its expressed biological activity. "Substantially purified" is defined as a composition in which the protein or peptide forms the major component of the composition, such as constituting of 50% or more. Typically, an isolated polypeptide is purified to at least 90% of the total protein (by mole fraction) in a sample. Preferably, the isolated polypeptide is 100% of the total protein in a sample (by mole fraction).

The evaluation of the degree of purification is done, for example, by determination of the specific activity of an active fraction in comparison to the initial crude extract and by SDS-PAGE analysis of the size and number of polypeptide band. The degree of purity is assessed as a "-fold purification number". Various techniques suitable for protein purification include precipitation with ammonium sulfate, polyethylene glycol (PEG) and antibodies followed by centrifugation, chromatography steps such as ion exchange, gel filtration, reverse phase, hydroxylapatite and affinity chromatography. The most purified state is not always a requirement and partially purified products may have advantages in total recovery of protein product, or in maintaining the activity of an expressed protein.

Immobilized metal ion adsorption chromatography (IMAC) can be used to purify proteins and peptides with polyhistidine tags. Briefly, a gel is first charged with divalent metal ions to form a chelate (Sulkowski, Trends in Biochem. 3:1-7, 1985), histidine-tagged proteins are adsorbed to this matrix and are eluted by competitive elution, lowering the pH or using strong chelating agents. Purification of glycosylated proteins can be accomplished by lectin affinity chromatography and ion exchange chromatography (Methods in Enzymol., Vol. 182, "Guide to Protein Purification", M. Deutscher, (ed.), Acad. Press, San Diego, 1990, pp.529-39). Additional fusions of the proteins or peptides of interest with affinity tags may be constructed.

The isolated selenomethionine-labelled polypeptide may be then subjected to the crystallisation procedure.
The incomplete factorial method based on sampling of a large number of randomly selected, but generally probable crystallization conditions may lead to a successful combination of reagents that generate crystals (Carter et al. (1979) J. Biol. Chem. 254: 12219-12223). Implementation of this idea described 32 solutions for the initial crystallization trials, which cover a range of pH, salts and precipitants (Jancarik et al. (1991) J. Appl. Crystallogr. 24: 409). The technology has recently been expanded to a set of 70 solutions and to minimize the human error rate and to establish a high-throughput format the solution preparation has been programmed for an automated robotic station (Ealick (2000) Curr. Opin. Chem. Biol. 4, 495-499).
The successive automated grid searching (SAGS) method has been used to generate a series of solutions which continually refine the crystallization conditions of temperature, pH, salts and precipitant (Weber et al. (1988) J.Cryst. Growth 90: 318-324). Once a solution that could reproducibly grow crystals was determined, a seeding technique which greatly improved the quality of the crystals was developed.

Generally, the method for crystallization, which may be used with any protein to be crystallized, comprises : (a) combination of aqueous aliquots of the desired protein with either (i) aliquots of a salt solution with different salt concentrations or (ii) aliquots of a precipitant solution with different precipitant concentrations, optionally, each combined aliquot is used in a range of different pH values, (b) observation of combined aliquots for precrystal formations, and selection of salt, precipitant and pH combinations or, if no precrystal forms are produced, increasing the starting concentration of protein; (c) after optimal salt and precipitant concentrations are selected, repetition of step (a) with previously unselected solution in the presence of selected concentration; and (d) repetition of step (b) and step (a) until a crystal of desired quality is obtained.

In interest of time and labor the above described method can be automated. The preferred protein starting concentrations are between 10 and 20 mg/ml. The range of salt solution to begin analysis with is 0-2.5M NaCl. PEG 8000 is a preferred precipitant and the pH values are 4.5, 5.0, 5.5, 6.0, 6.5, 7.0, 7.5, 8.0, 8.5, and 9.0. Precrystallization forms include oils, birefringement precipitants, small crystals (<approximately 0.05 mm), medium crystals (approximately 0.05 to 0.5 mm) and large crystals (>approximately 0.5 mm). The preferred waiting time to obtain crystal structures is 48 hours, although weekly observations are suggested.
Typically, the X-ray diffraction data is obtained to a level of resolution, which can yield structural information. This resolution may vary according to the detail of structural information required. Preferably, the resolution is at least 6A, more preferably at least 5A, still more preferably at least 4, 3.5, 3.2, or 3 or 2. 5A.

The X-ray crystallography can involve multiwavelength anomalous diffraction analysis.

By "multi-wavelength anomalous diffraction" (MAD) is meant a crystallographic technique in which X-ray diffraction data are collected at several different wavelengths from a single crystal containing a heavy atom (or heavy atoms) with absorption edges near the energy of incoming X-ray radiation. The resonance between X-rays and electron orbitals leads to differences in X-ray scattering that provide a solution of the crystallographic phase problem for a particular polypeptide. A detailed discussion of MAD analysis can be found in Hendrickson, Trans. Am. Crystallogr. Assoc., 21:11 (1985); Hendrickson et al., EMBO J., 9:1665 (1990); and Hendrickson, Science, 4:91 (1991).

Multiwavelength anomalous diffraction (MAD) phasing methods are applied and should substantially shorten the time required for resolving the crystal structure of target proteins. MAD phasing requires one or more atoms with an X-ray absorption edge in the energy range around 1 Å. C, N, O, S and H have absorption edges far away from the accessible energy range and are therefore not generally useful for MAD phasing. However, atoms such as transition metals with atomic numbers 20-40 or >60, have accessible absorption edges. For instance, selenium can be artificially introduced into proteins expressed in mammalian cells by replacement of methionine residues with selenomethionine. The present invention uses preferably MAD phasing experiments for recombinantly expressed proteins (Hendrickson et al. (1990) EMBO J 9, 1665-1672).

X-ray diffraction experiments are carried out on selenomethionine-labeled recombinant proteins expressed from alphavirus expression or other specific viral expression vectors, in particular SFV, in various mammalian host cells. X-ray intensity data are measured at several different wavelengths at or near the absorption edge of the anomalous scattering atoms. Alteration in the intensities of the individual reflections are caused by changes in the wavelength and the differences in intensities can be used to directly calculate the phase angle for each reflection at synchrotron beamlines.

MAD phasing data are measured with the same sample, sources of systematic errors are removed and the resulting phase angles are more accurate. Because anomalous scattering remains strong at high resolution, the phase angles are more accurately determined. Furthermore, MAD phasing does not involve trial-and-error procedures and a single experiment ordinarily leads to an interpretable electron density map. Finally, because electron density maps calculated with MAD phases are typically of high quality, the initial model can be built faster and with fewer errors, resulting in more rapid convergence during least-squares refinement.

MAD phasing techniques may be particularly useful for large macromolecular structures, which often present the most severe lack of isomorphism during heavy-atom derivatization and crystal freezing. Selenomethionine incorporation works well for solving large MAD structures assuming that the anomalous scattering atoms can be located.

Other aspects and advantages of the present invention will be disclosed in the following experimental section, which should be regarded as illustrative and not limiting the scope of protection.

### Examples

### Example 1

Test of survival of adherent and suspension cultures of BHK-21 and CHO-K1 cells in selenomethionine containing medium.

Adherent BHK-21 or CHO-K1 cells are cultured in a rich culture medium consisting of a 1:1 mixture of Dulbecco's F-12 medium and Iscoves's modified Dulbecco's medium supplemented with 10% FCS and 4 mM glutamine. Prior to infection with recombinant SFV particles expressing GFP (Green Fluorescence Protein) the medium is replaced with selenomethionine-containing medium. Cells are cultured for various periods of time and increasing concentrations of selenomethionine (10, 25 and 50 mg / L) and their viability is monitored by microscopical examination and other viability assays. GFP expression is monitored by fluorescence on living cells.

BHK-21 and CHO-K1 cells adapted to growth in suspension culture in commercially available serum-free medium are transferred to medium containing selenomethionine and subjected to infection with SFV-GFP particles. The cell viabilty and expression of recombinant GFP are monitored at increasing selenomethionine concentrations at various times post-infection.

### Example 2

Expression of selenomethionine labelled recombinant proteins in mammalian cells.

Topologically different proteins are expressed from SFV vectors as follows: A cytoplasmic, well-expressed reporter gene, GFP or LacZ, a membrane associated protein, human cyclo-oxygenase 2 (hCOX2), a transmembrane receptor, human neurokinin-1 receptor (hNK1R) and a secreted protein, human secreted alkaline phosphatase (hSAP). Each protein is expressed in several mammalian cell lines, e.g. BHK-21, CHO-K1, HEK293 and C6 glioma cells, in the presence and absence of selenomethionine.

Because all constructs contain tags (histidine, biotin) for purification purposes, expression levels can be verified by Western blotting also for proteins without available antibodies. Comparisons are made between expression levels in selenomethionine-containing medium and conventional medium.

Expression studies are initially performed in adherent cells before proceeding into suspension cultures in a small scale.

Large-scale production of well-expressed recombinant proteins is conducted to obtain material in sufficient quantities for purification and crystallization attempts.

## Claims

1. A method for producing labelled, recombinant polypeptides in mammalian host cells comprising the use of alphavirus or other specific viral expression vectors.

2. A method according to claim 1, wherein labelled, recombinant polypeptides are amino acid-labelled polypeptides, in particular selenomethionine-labelled recombinant proteins.

3. A method for producing selenomethionine labelled recombinant polypeptides, according to one of the preceding claims, comprising : a) transfecting mammalian cells with an alphavirus or other specific viral vector comprising a nucleic acid sequence encoding a polypeptide, b) culturing the cells in a selenomethionine-containing growth medium under conditions allowing incorporation of at least one selenomethionine residue into the polypeptide and, c) optionally, isolating the obtained polypeptide from the cell or the growth medium, said polypeptide being labelled.

4. A method according to any one of the preceding claims, wherein the alphavirus vector is a nucleic acid molecule comprising a recombinant alphavirus genome, which includes a target nucleic acid sequence encoding a selected polypeptide, the said molecule is a plasmid or a linear fragment, in the form of DNA or RNA, single- or double-stranded.

5. A method according to any one of the preceding claims, wherein the alphavirus vector corresponds to alphavirus particles comprising a nucleic acid molecule as described in claim 4.

6. A method according to any one of the preceding claims, wherein the origin of the alphavirus vector is chosen in the group consisting of Aura virus, Babanki virus, Barmah Forest virus, Bebaru virus, Buggy Creek virus, Chikungunya virus, Eastern Equine Encephalitis virus, Everglades virus, Fort Morgan virus, Getah virus, Highlands J virus, Kyzylagach virus, Mayaro virus, Middelburg virus, Mucambo virus, Ndumu virus, O'Nyong-Nyong virus, Pixuna virus, Ross River virus, Sagiyama virus, Semliki Forest virus, Sindbis virus, South African Arbovirus No. 86, Una virus, Venezuelan Equine Encephalitis virus, Western Equine Encephalitis virus and Whataroa virus.

7. A method according to any one of the preceding claims, wherein the alphavirus vector is derived from Semliki Forest virus (SFV), Sindbis virus (SIN) or Venezuelan Equine Encephalitis virus (VEE).

8. A method according to any one of the preceding claims 1 to 5, wherein the other specific viral expression vector is derived from Adenovirus or HSV.

9. A method according to any one of the preceding claims, wherein the polypeptide of interest is a membrane protein, either a partial membrane protein or an integral membrane protein, a nuclear, a cytoplasmic or a secreted protein.

10. A method according to any one of the preceding claims, wherein the alphavirus vector is or comprises a nucleic acid molecule or construct which comprises the sequence of a recombinant alphavirus genome, said recombinant alphavirus genome (i) being deficient for at least one alphavirus structural protein coding gene, (ii) comprises alphavirus non-structural protein coding sequences and regulatory sequences, and (iii) comprises a nucleic acid sequence coding the polypeptide.

11. A method according to any one of the preceding claims, wherein the alphavirus vector comprises a nucleic acid sequence encoding at least one, preferably all of the non-structural proteins nsP1-4 (including functional equivalents or derivatives thereof) and lack at least one functional nucleic acid encoding a structural alphavirus protein selected from C, E1, E2, E3 and 6K.

12. A method according to any one of the preceding claims, wherein the polynucleotide sequence encoding the polypeptide is located downstream of the non-structural coding sequences.

13. A method according to any one of the preceding claims, wherein the nucleic acid sequence encoding the polypeptide of interest is a heterologous RNA.

14. A method according to any one of the preceding claims, wherein the mammalian cells are blood cells, epithelial cells, endothelial cells, fibroblasts, hepatocytes, neurons, or glial cells.

15. A method according to any one of the preceding claims, wherein the mammalian cells are established as cell lines.

16. A method according to any one of the preceding claims, wherein the mammalian cells are human or rodent cells.

17. A method according to any one of the preceding claims, wherein the mammalian cells are BHK-21, CHO-K1, HEK293 or C6 glioma cells.

18. A method according to any one of the preceding claims, wherein the mammalian cells, in particular any population of human, murine or rat cells, comprise viral vector expressing systems as defined in any one of the preceding claims.

19. A method according to any one of the preceding claims 3 to 18, wherein the selenomethionine-containing growth medium typically contains between 1 and 100 mg/L, preferably 5 to 60 mg/L, and most preferably 7 to 55 mg/L of selenomethionine.

20. A method according to any one of the preceding claims, further comprising the following steps: d) forming a crystal of the polypeptide and, e) performing X-ray crystallography on the crystal, wherein the incorporated label, in particular the selenomethionine residue, is used to determine the structure of the polypeptide.

21. A method according to the preceding claim, wherein the method for crystallization comprises the following steps : (a) combination of aqueous aliquots of the desired protein with either (i) aliquots of a salt solution with different salt concentrations or (ii) aliquots of a precipitant solution with different precipitant concentrations, optionally, each combined aliquot is used in a range of different pH values, (b) observation of combined aliquots for precrystal formations, and selection of salt, precipitant and pH combinations or, if no precrystal forms are produced, increasing the starting concentration of protein; (c) after optimal salt and precipitant concentrations are selected, repetition of step (a) with previously unselected solution in the presence of selected concentration; and (d) repetition of step (b) and step (a) until a crystal of desired quality is obtained.

22. A method according to any one of claims 20 to 21, wherein the X-rays the X-ray diffraction data is obtained to a level of resolution which can yield structural information, preferably, the resolution is at least 6A, more preferably at least 5A, still more preferably at least 4, 3.5, 3.2, or 3 or 2. 5A.

23. A method according to any one of the claims 20 to 22, wherein the X-ray crystallography involves multiwavelength anomalous diffraction analysis.

24. A method for structure determination of a polypeptide comprising the method as defined in any one of the claims 20 to 23.

25. A kit for use in a method according to any one of the preceding claims, comprising a mammalian cells and/or a recombinant vector and/or selenomethionine-containing growth medium, as defined in any one of the preceding claims.

26. A use of a recombinant vector, mammalian cells, or selenomethionine-containing growth medium, as defined in one of the preceding claims, in a method for obtaining structural data on a protein of interest.
